# FASCICULE DE BREVET EUROPEEN

(11) **EP 1 156 840 B1**
(45) Date de publication et mention de la délivrance du brevet: **14.01.2004**
(21) Numéro de dépôt: 00903939.7
(22) Date de dépôt: 28.02.2000
(51) Int. Cl.: A61M 1/34

(54) **TUBULURE POUR L'EPURATION EXTRACORPORELLE DU SANG ET UTILISATION DE CETTE TUBULURE**
LEITUNGSSYSTEM FÜR DIE EXTRAKORPORALE BLUTREINIGUNG UND VERWENDUNG DESSELBEN
TUBING FOR EXTRACORPORAL PURIFICATION OF THE BLOOD AND USE THEREOF

(30) Priorité: 02.03.1999 FR 9902732
(43) Date de publication de la demande: 28.11.2001
(73) Titulaire: Infomed S.A., 1227 Genève (CH)
(72) Inventeur: FAVRE, Olivier, CH-1208 Genève (CH)
(74) Mandataire: Micheli, Michel-Pierre
(86) Numéro de dépôt international: PCT/IB2000/000216
(87) Numéro de publication internationale: WO 2000/051664

(56) Documents cités:
- EP-A- 0 826 383
- EP-A- 0 834 329
- WO-A-90/15631
- US-A- 4 552 721
- US-A- 4 888 004
- US-A- 5 762 782

## Description

La présente invention se rapporte à une tubulure pour l'épuration extracorporelle du sang d'un être humain ou d'un animal à sang chaud, comprenant un conduit de circulation extracorporelle en boucle ouverte présentant deux parties, l'une d'extraction du sang à épurer, l'autre de retour du sang épuré, destinées à être raccordées à des moyens d'épuration, au moins un conduit pour relier au moins une desdites parties à une source d'une solution de substitution, un piège à bulles situé le long de ladite boucle, un conduit d'évacuation du produit rejeté par lesdits moyens d'épuration, muni d'un segment conformé pour servir de corps de pompe destiné à être mis en prise avec une pompe péristaltique et des moyens de raccordement pour relier l'une à l'autre, de façon amovible, les extrémités respectives de ladite tubulure de circulation extracorporelle en boucle ouverte pour former une boucle fermée. Cette invention se rapporte aussi à une utilisation de cette tubulure.

Ce type de tubulure est utilisé notamment pour des malades souffrant d'insuffisance rénale, nécessitant d'éliminer une ou plusieurs substances nocives et/ou de voir leur poids contrôlé au cours du temps. Les dispositifs qui existent pour traiter les patients atteints de défaillance rénale sont généralement conçus pour être utilisés par des professionnels en milieu hospitalier. Dans le cas d'une défaillance chronique le malade doit se rendre régulièrement, typiquement trois fois par semaine, en milieu hospitalier spécialisé, au détriment de sa qualité de vie.

Dans le cas d'une défaillance aiguë associée à un état de crise et limitée dans le temps, le malade est traité en soins intensifs par un personnel soignant non spécialisé dans ce genre de thérapie.

Les principales méthodes de traitement sont l'hémodialyse qui consiste en un échange ionique par diffusion entre le sang et un liquide appelé dialysat et l'hémofiltration qui agit par convection en filtrant mécaniquement le sang. Les deux méthodes utilisent une membrane artificielle comme filtre semi-perméable.

Ce processus de traitement comporte trois phases, comprenant une phase de préparation de la tubulure, une phase de traitement proprement dite et une phase de rangement de cette tubulure. La préparation comporte deux séquences qui sont souvent distinctes, l'amorçage du circuit qui permet de remplir les tubulures et le filtre en éliminant l'air, et le rinçage qui nettoie les tubulures et le filtre en vue de réaliser le traitement. Après ce traitement, selon que l'on souhaite ou non réutiliser la tubulure, on la désinfecte en laissant à l'intérieur un désinfectant qui évite le développement de bactéries, ou on se contente de la vider avant de la jeter.

Pour préparer une tubulure de l'état de la technique en vue d'effectuer une opération d'épuration extracorporelle du sang, on connecte l'extrémité d'extraction du sang de la tubulure à un réservoir de solution et l'extrémité de retour du sang de cette tubulure à un collecteur de liquide. On fait ensuite circuler cette solution au moyen de la pompe d'extraction du sang, du réservoir de solution au collecteur de liquide.

Ce processus de préparation entraîne plusieurs inconvénients. Il nécessite l'utilisation de sacs supplémentaires qui doivent ensuite être éliminés en observant les prescriptions de sécurité. A chaque changement, ces sacs doivent être ouverts et fermés. Il entraîne donc un important travail de manipulation. De plus, la tubulure n'est pas réutilisable, en raison de la complexité des opérations de vidange et de nettoyage que nécessiterait cette réutilisation.

Les phases de préparation et de rangement du dispositif sont donc complexes à réaliser et prennent un temps important. Durant le traitement la purge de la tubulure et les changements des réservoirs de liquide prennent également du temps et sont en outre difficiles à réaliser par le patient lui-même.

On a déjà proposé, notamment dans le US 4 888 004 ainsi que dans le WO 90/15631, des tubulures comportant des moyens de liaison amovible entre les deux extrémités de la boucle ouverte pour former une boucle fermée, permettant de faire circuler une solution de rinçage ou pour amorcer le circuit avant le traitement d'un patient. Bien que ces documents se rapportent à des circuits comportant notamment un piège à bulles, destiné à séparer la phase gazeuse de la phase liquide, aucun d'eux ne permet d'établir une communication directe entre le piège à bulles et le réservoir de récupération. En effet dans le WO 90/15631, le conduit reliant le piège à bulles au réservoir de récupération est en prise avec une pompe péristaltique, de sorte que la phase gazeuse ne peut pas s'évacuer librement. Il en est évidemment de même d'un éventuel trop-plein, puisque le débit à travers le conduit d'évacuation est contrôlé par la pompe péristaltique. Dans le US 4 888 004, aucun conduit ne relie le piège à bulles au réservoir de récupération.

Le but de la présente invention est de remédier au moins en partie aux inconvénients susmentionnés.

A cet effet, cette invention a tout d'abord pour objet une tubulure du type mentionné précédemment, selon la revendication 1.

Cette invention a également pour objet une utilisation de la tubulure, telle que définie par la revendication 6.

La tubulure objet de cette invention est particulièrement simple d'emploi de sorte qu'elle rend le traitement accessible au malade qui peut se soigner lui-même à domicile, ou dans un centre spécialisé, dans la mesure où elle permet de réduire au minimum toute intervention au cours des trois phases du processus de traitement. Elle a en effet été conçue en vue d'une gestion automatique, ou quasi automatique de toutes ses phases de fonctionnement aussi bien avant, pendant, qu'après le traitement d'épuration du sang. Elle permet également, de ce fait, d'être utilisée en milieu hospitalier par du personnel non spécialisé dans ce genre de traitement, notamment par le personnel des soins intensifs.

Grâce à sa conception, cette tubulure peut non seulement servir à évacuer librement le gaz, mais également le trop-plein de liquide, tout en permettant de détecter la présence indésirable de sang qui s'écoulerait accidentellement à travers le conduit d'évacuation. Ces avantages vont dans le sens d'une plus grande sécurité du patient, d'une diminution des risques infectieux et d'une simplicité de mise en oeuvre.

Les dessins annexés illustrent, très schématiquement et à titre d'exemple, une forme d'exécution de la tubulure, ainsi qu'un mode d'utilisation de cette tubulure, objets de la présente invention.
La figure 1 illustre le schéma d'un dispositif pour l'hémofiltration ou la plasmaphérèse dans lequel est utilisée une tubulure selon cette forme d'exécution;
la figure 2 illustre une procédure de préparation de la tubulure à un processus d'épuration;
la figure 3 illustre une procédure de rangement de cette tubulure;
les figures 4a-4c illustrent un détail à plus grande échelle des moyens de raccordement vus respectivement en élévation latérale (4a), tourné de 90° autour d'un axe transversal (4b) et en élévation latérale assemblés aux extrémités de la tubulure (4c) ;
la figure 5 illustre une variante de la tubulure permettant de connecter le système de circulation intracorporelle du patient au conduit de circulation extracorporelle au moyen d'un accès vasculaire unique.

La tubulure objet de la présente invention se présente comme un conduit en boucle ouverte qui comporte une tubulure d'extraction 1 séparée d'une tubulure de retour 2 par un filtre 6 ou tout autre moyen d'épuration approprié. Sur la tubulure de retour 2 sont placés un détecteur de bulles d'air 3 et un clamp 4. Une pompe péristaltique 5 sert principalement à extraire le sang du patient et à le faire circuler dans la boucle ouverte lorsque ses deux extrémités sont connectées en deux portions distinctes de la circulation sanguine intracorporelle du patient. Un piège à bulles 7 permet d'une part d'arrêter les bulles d'air en déplacement dans la boucle de circulation extracorporelle et d'autre part d'introduire et/ou d'extraire des fluides dans cette boucle de circulation 1, 2.

Cette tubulure comporte encore un conduit d'extraction du liquide filtré 28, associé à une pompe d'extraction du produit rejeté 19, à un détecteur de fuite de sang 14 et un collecteur 15 de ce produit rejeté. Un capteur de pression 25, un capteur de conductivité électrique 26, un capteur de température 27 et un capteur d'équilibre acido-basique 24 seront encore avantageusement associés à ce conduit d'extraction 28. Ces capteurs pourront encore être associés à des moyens d'affichage (non représentés) et à des moyens de calcul (non représentés) utilisés pour la gestion du processus d'épuration.

La tubulure selon l'invention comporte encore un conduit 29, permettant de relier une source de solution de substitution 16 à la boucle de circulation extracorporelle 1, 2. Ce conduit 29 est associé à une pompe de circulation 17 commandant le débit de solution de substitution dans un conduit 30 qui relie le conduit 29 à la tubulure de retour 2 de la boucle de circulation extracorporelle. Un second conduit 31 relie le conduit 29 à la tubulure d'extraction 1 de la boucle ouverte de circulation extracorporelle. De préférence, ce second conduit 31 est en deux parties assemblées par un connecteur 44 dont le rôle est expliqué dans la description de la procédure de nettoyage. Une pompe 18 associée à ce conduit 31 contrôle le débit de la solution de substitution qui s'écoule vers la tubulure d'extraction 1. En variante, on pourrait utiliser une seule pompe associée au conduit 29 et un répartiteur de débit disposé à l'embranchement des conduits 30 et 31.

Un conduit de liaison 8 relie la boucle de circulation 1, 2 à la tubulure d'extraction 28. Ce conduit de liaison 8 part du piège à bulles 7 et aboutit au détecteur de sang 14. Grâce à ce conduit de liaison 8, la boucle de circulation est rendue étanche à l'air ambiant tout en permettant d'évacuer les bulles qu'elle renferme, généralement sous forme d'un mélange des phases liquide et gazeuse. Ce conduit fait, de préférence, partie intégrante de la tubulure et peut donc être stérilisé avec elle. Pour permettre à l'air de s'échapper vers l'atmosphère le collecteur 15 peut être muni d'une soupape de surpression 48, ou le collecteur peut être une enceinte non étanche. Une poche souple et étanche vide en début de traitement est également une forme de réalisation possible du collecteur 15.

Une valve anti-retour 49, disposée le long du conduit de liaison 8, ne permet l'écoulement de liquide dans ce conduit que dans le sens allant de la boucle de circulation 1, 2, vers le conduit d'évacuation 28 et empêche ainsi que le produit rejeté atteigne le piège à bulles 7 en cas d'erreur de manipulation. Un clamp 47 permet de contrôler le débit dans ce conduit de liaison 8, de fermer cette connexion.

Ce conduit de liaison 8 évite l'entrée d'air dans,la boucle de circulation 1, 2 en cas d'erreur de manipulation, puisqu'il plonge dans le détecteur de sang 14 qui est normalement rempli d'ultrafiltrat. Si ce détecteur détecte la présence de sang, il émet un signal destiné à arrêter les pompes 5, 17, 18 et 19. De plus, ce conduit de liaison 8 évite toute fuite de liquide vers l'extérieur.

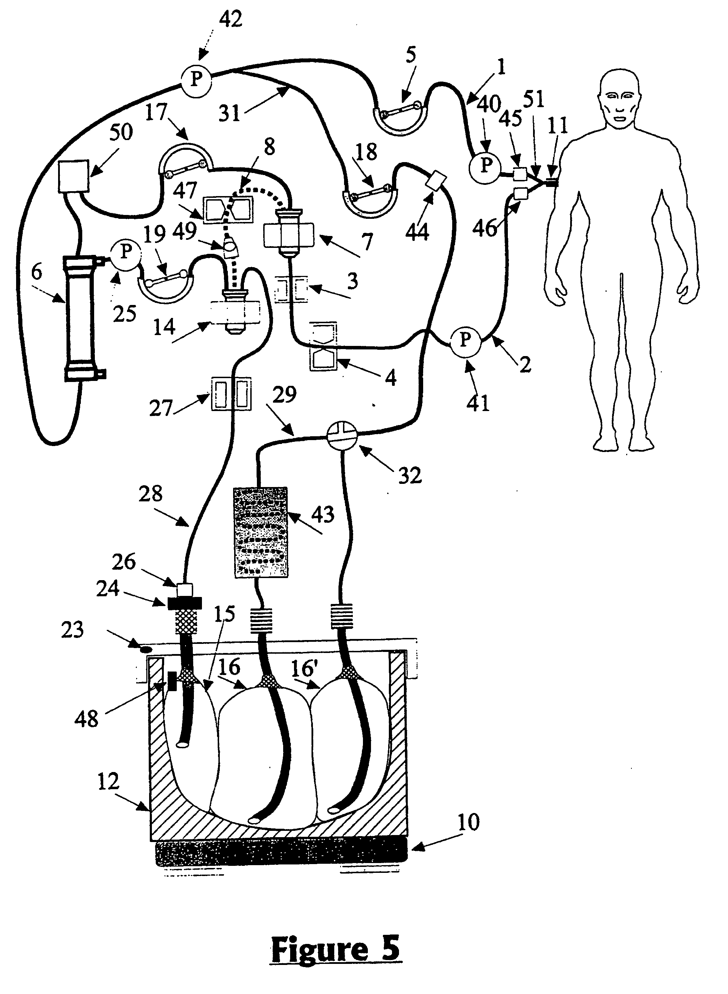

Dans la variante illustrée sur la figure 5, un réservoir tampon étanche 50 est ajouté sur le tronçon de la boucle de circulation existant entre les pompes 5 et 17 cette dernière étant placée sur la tubulure de retour 2, un raccord en forme de Y 51 relie la tubulure d'extraction 1 à la tubulure de retour 2, et la boucle de circulation extracorporelle ainsi formée à un accès.unique de la circulation sanguine du patient, le raccord 11 devenant un bouchon dont la forme peut autoriser, ou non, la circulation du liquide à l'extérieur du raccord 51. La liaison entre la source de solution 16 et la boucle de circulation extracorporelle est placée sur la tubulure d'extraction 1, elle peut également être placée sur la tubulure de retour 2 ou sur les deux simultanément.

De préférence, l'enceinte de récupération 15 et une ou plusieurs sources de solution de substitution 16, 16' sont constituées par des poches souples disposées dans un ou plusieurs conteneurs 12, de préférence rigide. L'information relative à ce conteneur 12 peut avantageusement être enregistrée et affichée par un organe 23. Le conteneur utilisé et différentes variantes possibles correspondent de préférence au conteneur décrit dans le FR 2 782 916 auquel on pourra se référer pour plus de détails.

Un dispositif de pesage 10 peut être associé au conteneur 12 pour mesurer les quantités de liquide échangées. A titre de variantes ces moyens de pesage peuvent être remplacés par des moyens de mesure de volume ou de niveau de liquide ou encore par des capteurs de débits qui donnent une information équivalente.

Différents capteurs peuvent compléter le dispositif, notamment des capteurs de déplacement des pompes (non représentés), des capteurs de pression (40, 41, 42, 25), ou encore un ou plusieurs capteurs de température 27.

Les différents capteurs associés à la tubulure objet de l'invention sont reliés à des moyens de calcul (non représentés) qui comportent essentiellement des moyens électroniques et informatiques permettant notamment de recueillir et de traiter les informations pour agir sur différents organes.

On peut encore prévoir un moyen de chauffage 43 du sang et/ou de la solution de substitution et/ou de désinfection, lequel moyen de chauffage peut être disposé à un endroit quelconque du dispositif.

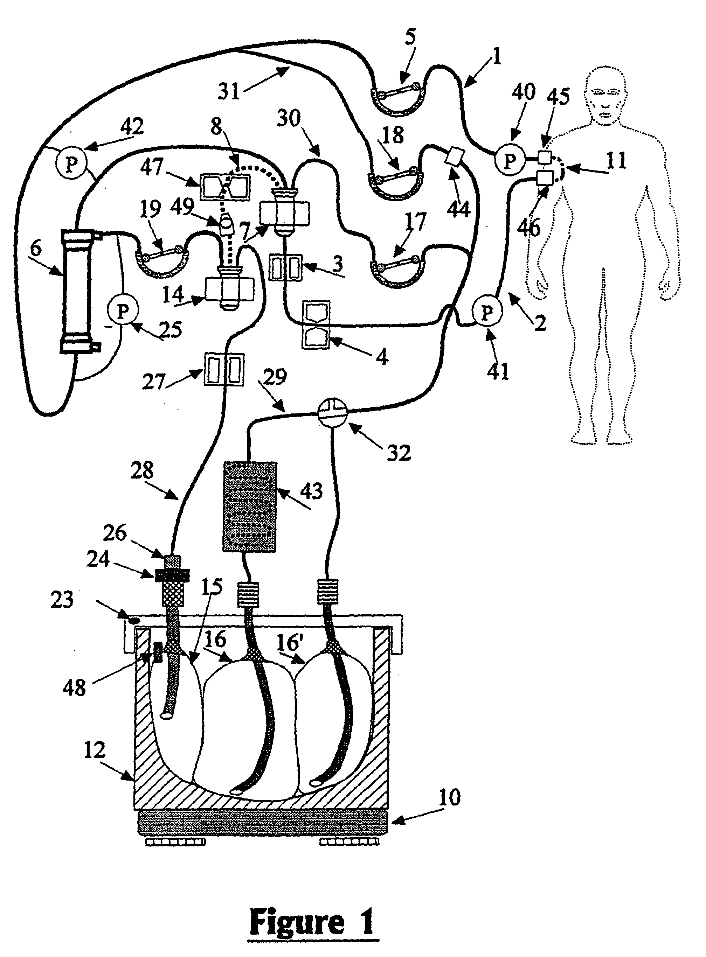

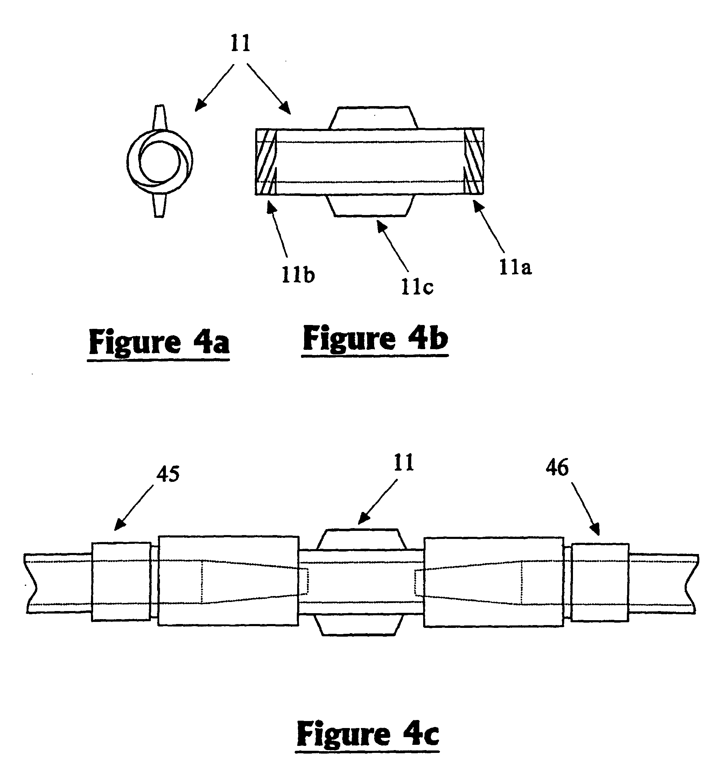

Après mise en place de la tubulure sur l'appareil selon la figure 1, l'amorçage de la tubulure consiste tout d'abord, si ce n'est déjà fait, à relier l'une à l'autre les deux extrémités de la boucle ouverte à l'aide du conduit de raccordement 11. Ce conduit de raccordement 11, montré plus en détail aux figures 4a, 4b, 4c possède deux connexions 11a, 11b qui sont identiques à celles qui permettent de relier la tubulure à la circulation sanguine du patient par l'intermédiaire d'un cathéter ou d'un autre élément de connexion (non représenté), de sorte que les extrémités ouvertes 45, 46 de la tubulure 1, 2 peuvent ainsi être adaptées indifféremment aux cathéters ou au conduit de raccordement 11. Le volume interne de ce conduit de raccordement 11 est quasi nul pour éviter les pertes de liquide lors de la connexion de cette tubulure au patient, auquel sont fixées les extrémités 45 et 46. De préférence, le conduit de raccordement 11 est donc conçu pour que les deux extrémités de la boucle ouverte de circulation 1, 2 soient placées bout à bout. Avantageusement, ce conduit de raccordement 11 est réalisé en matière plastique et est stérilisé en même temps que le reste de la tubulure avec laquelle il est livré. Une partie élargie 11c peut être prévue pour faciliter le maintien du raccord pendant les opérations de connexion et déconnexion.

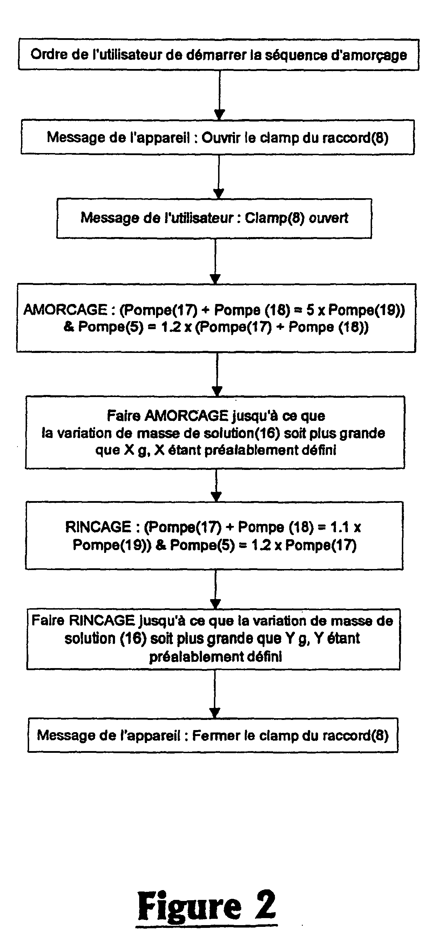

Selon une procédure de préparation préférée représentée sur la figure 2, l'amorçage consiste à faire tourner les pompes d'extraction du sang 5, de prédilution 17 et/ou de postdilution 18 de manière à remplir les tubulures 1, 2 et le filtre associé 6, la pompe d'extraction du produit rejeté 19 est également actionnée pour aider à purger la boucle de circulation 1, 2, mais à un débit plus faible. Durant la phase de rinçage, le débit d'extraction du filtrat de la pompe 19 est maintenu à une valeur inférieure, éventuellement nulle, à la somme des débits de prédilution et postdilution des pompes 17 et 18, la pompe 5 d'extraction du sang fournissant un débit suffisant pour ne pas freiner celui des pompes 17 et 18. La tubulure est ainsi remplie de liquide durant toute l'opération, le trop-plein, s'évacuant à travers le conduit 8, à un débit équivalent à la différence entre celui retiré (filtrat) et celui ajouté (prédilution et postdilution).

L'arrêt de ces deux opérations d'amorçage et de rinçage se fait normalement automatiquement lorsque des masses de solution de substitution préalablement déterminées ont été échangées. L'arrêt peut exceptionnellement être provoqué par l'utilisateur lui-même ou une alarme, par exemple lorsque des bulles d'air sont détectées par le détecteur 3 durant le rinçage.

A la fin du rinçage, une opération consistant à activer la pompe à sang 5 et la pompe de filtrat 19 simultanément permet d'abaisser le niveau de liquide dans le piège à bulles 7. Le clamp 47 est ensuite fermé en vue de réaliser l'épuration du sang.

Dans le cas de la tubulure illustrée à la figure 5, l'amorçage et le rinçage suivent le même principe que précédemment décrit. La pompe 18 injecte du liquide à un débit supérieur au débit d'extraction fourni par la pompe 19, le surplus étant éliminé à travers le conduit 8, les pompes 5 et 17 placées sur le conduit de circulation extracorporelle étant commandées de manière maintenir une pression raisonnable dans l'ensemble du circuit, évitant ainsi de détériorer ce dernier. Cette commande se base, de manière préférée, sur les indications des capteurs de pression 40, 41, 42, 25.

Un avantage supplémentaire de la fermeture de la boucle de circulation 1, 2 par le conduit de raccordement 11 est de rendre possible le contrôle des capteurs de pression pendant le rinçage. La fermeture de cette boucle de circulation 1, 2 par des éléments non déformables, permet de vérifier que les pressions lues par les capteurs 40 et 41 sont identiques et qu'elles augmentent lorsque la pompe 18 et/ou la pompe 17 tournent, alors que les autres sont à l'arrêt. On peut vérifier que la pression lue par le capteur 25 augmente proportionnellement au débit de la pompe d'extraction du filtrat 19 pour un débit constant et non nul de la pompe d'extraction du sang 5. On peut aussi vérifier que la pression lue par le capteur 42 augmente proportionnellement au débit de la pompe 5 les autres étant arrêtées.

Les variations de pressions mesurées en fonction du déplacement de ces pompes sont comparées à une courbe de référence enregistrée dans une mémoire lors d'une procédure de calibration de l'appareil. La comparaison des mesures avec la courbe de référence correspondante permet de déterminer si la réponse des capteurs de pression est correcte en appliquant des méthodes classiques de traitement du signal. Notons que l'on vérifie également ici que les pompes sont occlusives et que les tubulures utilisées sont celles qui correspondent au traitement qui sera réalisé, ces conditions étant nécessaires pour obtenir une réponse satisfaisante des capteurs de pression.

Pour purger l'air de la boucle de circulation 1, 2, au cours de la phase d'épuration du sang, on ouvre le clamp 47 qui est normalement fermé durant cette phase d'épuration. Cette ouverture peut être commandée automatiquement ou manuellement. Le clamp 4 est fermé pour éviter que des bulles ne parviennent au patient, puis on entame une procédure automatique qui fait tourner la pompe 17, le surplus de liquide injecté remplaçant progressivement les bulles d'air qui sont éliminées à travers le conduit de liaison 8. L'opérateur arrête le processus lorsqu'il constate que le niveau de liquide dans le piège à bulles 7 est suffisant pour poursuivre l'épuration du sang. Le conduit de liaison 8 est ensuite fermé et le clamp 4 est ouvert à nouveau pour laisser le sang circuler. Si la circulation du sang au moyen de la tubulure est évidente dans le cas classique illustré par la figue 1, il convient de préciser comment elle est assurée dans le cas ou un accès unique à la circulation sanguine du patient est réalisé. Lors du rinçage, le réservoir tampon 50 est rempli de liquide. Au début de la phase d'épuration du sang, le dispositif commence par retourner ce liquide au patient en activant la pompe 17, les autres étant arrêtées. Après qu'un certain volume de liquide ou un certain temps se soient écoulés la pompe 17 est arrêtée et les pompes 5, 18 et 19 sont actionnées, comprimant ainsi l'air compris entre le sang contenu dans le réservoir 50 et le sommet de ce dernier, jusqu'à ce qu'une pression mesurée par le capteur 42 soit supérieure à une limite connue du système. L'épuration est ainsi réalisée en assurant un niveau de remplissage suffisant, pour assurer la continuité de la circulation de sang, du réservoir 50 à chaque cycle. Ce cycle en deux phases est reproduit jusqu'à la fin de la séance d'épuration.

La condition de réussite de l'opération est que le volume de liquide retiré à chaque cycle du réservoir 50 par la pompe 17 soit inférieur au volume du réservoir lui-même.

Un problème important des traitements d'épuration extracorporelle est le coût financier et écologique des éléments consommables associés à chaque traitement.

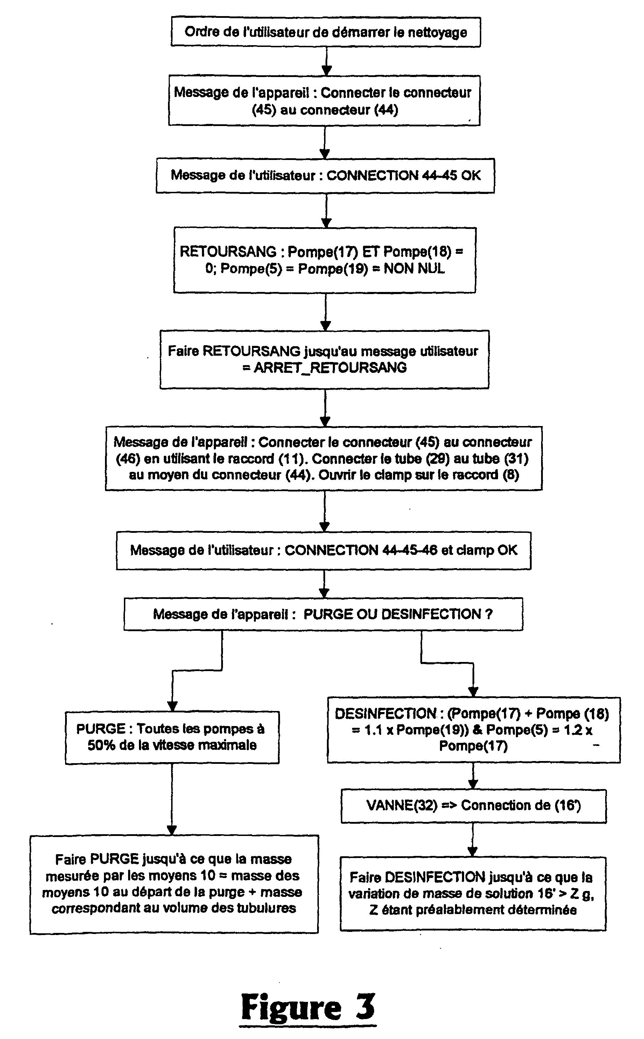

L'utilisation de la tubulure selon l'invention permet de la conserver sur l'appareil en effectuant la phase de rangement qui sera décrite en relation avec la figure 3.

On commence par débrancher l'extrémité 45 de la partie d'extraction du sang 1 de la tubulure du cathéter, on sépare le conduit de prédilution 31 au niveau du connecteur 44 et on relie l'extrémité 45 à la partie du conduit de prédilution 31 qui communique avec la source de solution 16 ou 16'. On pompe alors la solution à l'aide de la pompe 5 de manière à retourner le sang vers le patient.

Une fois cette opération terminée, on débranche les extrémités 45 et 46 de la boucle de circulation pour les réunir à l'aide du conduit de raccordement 11 positionné comme lors de l'amorçage de la tubulure. Les deux parties du conduit de prédilution 31 sont à nouveau réunies au raccord 44. On ouvre le clamp 47 qui ferme le conduit de liaison 8 puis, on effectue la vidange complète ou la désinfection de la tubulure. La désinfection peut être suivie d'un rinçage avec une autre solution ou d'une vidange. En variante, si l'on souhaite effectuer uniquement une vidange, on peut débrancher l'extrémité 46 du patient et la placer de manière à ce que son contenu se vide, via une ouverture non représentée, directement dans le conteneur 15, évitant ainsi de devoir toucher aux autres partie de la tubulure.

La vidange du circuit s'effectue en faisant tourner toutes les pompes, de préférence avec les clamps ouverts, la totalité du liquide se retrouvant ainsi dans le collecteur 15 et étant mesurée par la balance 10. On arrête la vidange lorsque le poids mesuré par la balance indique que les débits y relatifs sont nuls. La désinfection est réalisée en orientant une vanne 32 vers un compartiment par exemple 16' qui peut contenir un liquide de désinfection, par exemple de l'acide ou une solution saline concentrée, que l'on fait circuler à l'aide des pompes dans des conditions identiques à celles de rinçage de la boucle de circulation 1, 2, de manière à s'assurer que la totalité de cette boucle est mise en contact avec le désinfectant. Cette étape se termine après qu'un certain temps ou un certain volume se soit écoulé. Pour effectuer un rinçage après la désinfection, la vanne 32 est orientée vers le compartiment 16, qui contient la solution de rinçage, puis les pompes sont actionnées dans les mêmes conditions que précédemment. Le rinçage se termine selon les mêmes critères que la désinfection.

Différentes précautions doivent être prises pour effectuer ces opérations et s'assurer que le fluide utilisé pour la désinfection est correctement rincé avant de commencer l'opération d'épuration. Le contrôle est effectué de préférence, d'une part au niveau de la vanne 32, dont le déplacement est contrôlé au moyen d'au moins un capteur (non représenté), et d'autre part, grâce au capteur d'équilibre acido-basique 24 et/ou de conductivité électrique 26 placés sur la tubulure d'extraction 28. La mesure est effectuée lorsque la pompe d'extraction 19 est actionnée, la valeur obtenue étant alors représentative de celle du liquide circulant dans la boucle de circulation 1, 2. Cette valeur mesurée peut ensuite être comparée avec une valeur de référence mémorisée.

## Revendications

1. Tubulure pour l'épuration extracorporelle du sang d'un être humain ou d'un animal à sang chaud, comprenant un conduit de circulation extracorporelle en boucle ouverte présentant deux parties, l'une d'extraction du sang à épurer (1), l'autre de retour du sang épuré (2), destinées à être raccordées à des moyens d'épuration (6), au moins un conduit (29, 30, 31) pour relier au moins une desdites parties (1, 2) à une source (16, 16') d'une solution de substitution, un piège à bulles (7) situé le long de ladite boucle; un conduit d'évacuation du produit rejeté (28) dans une enceinte de récupération (15) par lesdits moyens d'épuration, muni d'un segment conformé pour servir de corps de pompe destiné à être mis en prise avec une pompe péristaltique et des moyens de raccordement (11) pour relier l'une à l'autre, de façon amovible, les extrémités respectives (45, 46) de ladite tubulure de circulation extracorporelle en boucle ouverte (1, 2) pour former une boucle fermée, ledit conduit d'évacuation du produit rejeté (28) comportent, en aval dudit segment conformé pour servir de corps de pompe, un détecteur de sang (14), **caractérisé en ce qu'**un conduit de liaison (8) s'étend entre l'extrémité aval dudit segment conformé pour servir de corps de pompe et ledit piège à bulles (7).

2. Tubulure selon la revendication 1, **caractérisée en ce qu'**elle comporte de plus un réservoir tampon (50) sur le tronçon de la boucle de circulation destinée à être situé en aval desdits moyens d'épuration.

3. Tubulure selon l'une des revendications précédentes, **caractérisée en ce que** ledit conduit de liaison (8) comporte des moyens (47) pour contrôler le débit à travers lui.

4. Tubulure selon l'une des revendications précédentes, **caractérisée en ce que** chacune desdites parties (1, 2) dudit élément de circulation est reliée à ladite source de solution de substitution (16, 16').

5. Tubulure selon l'une des revendications précédentes, **caractérisée en ce que** ledit conduit (29) pour relier au moins l'une desdites parties dudit conduit de circulation (1, 2) à ladite source de solution de substitution comporte un embranchement et des moyens de commutation (32) pour relier alternativement ladite partie à au moins deux enceintes (16; 16') de ladite solution.

6. Utilisation de la tubulure selon l'une des revendications précédentes, ledit conduit de circulation (1, 2) formant une boucle fermée, **caractérisée en ce qu'**on fait circuler du liquide dans ladite boucle de circulation (1, 2) fermée pour évacuer l'air qu'elle renferme par ledit conduit de liaison (8).

## Claims

1. Tubing for the extracorporal purification of the blood of a human being or a warm-blooded animal, comprising an open loop extracorporal circulation conduit having two portions, one for extraction of the blood to be purified (1), the other for return of the purified blood (2), adapted to be connected to purification means (6), at least one conduit (29, 30, 31) to connect at least one of said portions (1, 2) to a source (16,16') of a substitution solution, a bubble trap (7) located along said loop, an evacuation product conduit (28) for product rejected into a recovery chamber (15) by said purification means, provided with a segment adapted to serve as a pump body adapted to be connected with a peristaltic pump and connection means (11) removably to connect to each other the respective ends (45, 46) of said open loop extracorporal circulation tubing (1, 2) to form a closed loop, **characterized in that** said evacuation conduit (28) for rejecting product comprises, downstream of said segment adapted to serve as a pump body, a blood detector (14), a connection conduit (8) extending between the downstream end of said segment adapted to serve as a pump body and said bubble trap (7).

2. Tubing according to claim 1, **characterized in that** it moreover comprises a buffer reservoir (50) in the section of the circulation loop between the pumps 5 and 17.

3. Tubing according to one of the preceding claims, **characterized in that** said connection conduit (8) comprises means (47) to control the flow rate through it.

4. Tubing according to one of the preceding claims, **characterized in that** each of said portions (1, 2) of said circulation element is connected to said source (16,16') of substitution solution.

5. Tubing according to one of the preceding claims, **characterized in that** said conduit (29) for connecting at least one of said portions of said circulation conduit (1, 2) to said source of substitution solution, comprises a junction and switching means (32) alternatively to connect said portion to at least two chambers (16, 16') for said solution.

6. The use of tubing according to one of the preceding claims, said circulation conduit (1, 2) forming a closed loop, **characterized in that** the liquid is caused to circulate in said closed circulation loop (1, 2) to evacuate air which it contains, through said connection conduit (8).

## Patentansprüche

1. Leitungssystem für die extrakorporale Blutreinigung bei einem Menschen oder Warmblüter mit einer als offene Schleife ausgelegten Zirkulationsleitung, die zwei Abschnitte aufweist, einen für die Förderung des zu reinigenden Blutes (1), den anderen für die Rückführung des gereinigten Blutes (2), die dafür bestimmt sind, an Reinigungsorgane (6) angeschlossen zu werden, mit zumindest einer Leitung (29, 30, 31), um zumindest einen dieser Abschnitte (1, 2) mit einer Quelle (16, 16') von Austauschlösung zu verbinden, mit einer entlang dieser Schleife angeordneten Blasenfalle (7), mit einer Leitung für die Entleerung von Abfallprodukten (28), die durch diese Reinigungsorgane in ein Auffanggefäss (15) ausgeschieden werden, mit einem Segment versehen, das so gestaltet ist, dass es als Pumpenkörper dienen kann, um mit einer Schlauchpumpe in Eingriff zu kommen, sowie mit Verbindungselementen (11), um in lösbarer Weise die beiden Enden (45, 46) dieser offenschleifigen extrakorporalen Zirkulationsleitung (1, 2) miteinander zu verbinden und eine geschlossene Schleife zu bilden, wobei diese Leitung für die Entleerung von Abfallprodukten (28) strömungsabwärts von diesem als Pumpenkörper gestalteten Segment ein Blutspürgerät (14) aufweist, **dadurch gekennzeichnet, dass** eine Verbindungsleitung (8) sich zwischen dem Ende strömungsabwärts von diesem als Pumpenkörper gestalteten Segment und dieser Blasenfalle (7) erstreckt.

2. Leitungssystem nach Anspruch 1, **dadurch gekennzeichnet, dass** es weiterhin ein Puffergefäss (50) in dem Abschnitt der Zirkulationsschleife aufweist, der dafür bestimmt ist, strömungsabwärts von diesen Reinigungsorganen angeordnet zu werden.

3. Leitungssystem nach einem der vorangehenden Ansprüche, **dadurch gekennzeichnet, dass** diese Verbindungsleitung (8) Organe (47) zur Steuerung ihres Durchflusses enthält.

4. Leitungssystem nach einem der vorangehenden Ansprüche, **dadurch gekennzeichnet, dass** jeder der benannten Abschnitte (1, 2) dieses Zirkulationselements mit dieser Quelle von Austauschlösung (16, 16') verbunden ist.

5. Leitungssystem nach einem der vorangehenden Ansprüche, **dadurch gekennzeichnet, dass** diese Leitung (29) zur Verbindung zumindest eines dieser Abschnitte dieser Zirkulationsleitung (1, 2) mit dieser Quelle von Austauschlösung eine Verzweigung sowie Umschaltorgane (32) enthält, um diesen Abschnitt wechselweise mit zumindest zwei Behältern (16, 16') dieser Lösung zu verbinden.

6. Verwendung des Leitungssystems nach einem der vorangehenden Ansprüche, wobei diese Zirkulationsleitung (1, 2) eine geschlossene Schleife bildet, **dadurch gekennzeichnet, dass** Flüssigkeit durch diese geschlossene Zirkulationsschleife (1, 2) geschickt wird, um die Luft, die in ihr enthalten ist, durch diese Verbindungsleitung (8) zu ventilieren.
